Europäisches Patentamt

(19) European Patent Office

Office européen des brevets

(11) Publication number: **0 012 476**
**B1**

(12) # EUROPEAN PATENT SPECIFICATION

(45) Date of publication of patent specification: **25.08.82**

(51) Int. Cl.³: **C 02 F 3/28**

(21) Application number: **79200723.9**

(22) Date of filing: **04.12.79**

(54) A process and apparatus for anaerobic conversion of organic material in an aqueous medium.

(30) Priority: **08.12.78 NL 7811999**

(43) Date of publication of application:
**25.06.80 Bulletin 80/13**

(45) Publication of the grant of the patent:
**25.08.82 Bulletin 82/34**

(84) Designated Contracting States:
**BE DE FR GB IT LU SE**

(73) Proprietor: **STORK AMSTERDAM B.V.**
**2 Ketelstraat**
**NL-1021 JZ Amsterdam (NL)**

(72) Inventor: **van Drooge, Barend Lodewijk**
**48, Gerrit v.d. Veenstraat**
**NL-1077 EG Amsterdam (NL)**

(74) Representative: **van der Veken, Johannes Adriaan**
**et al,**
**3 & 4 Willem Witsenplein**
**NL-2596 BK The Hague (NL)**

(56) References cited:
AT - A - 337 624
DE - A - 1 931 777
DE - A - 2 728 585
FR - A - 376 442
GB - A - 1 390 981
US - A - 3 977 978

DE INGENIEUR, vol. 90, no. 40, October 1978, KIVI, pages 755—757 Den Haag, NL. R. J. ZOETEMEIJER: "Aspecten van de verzuring in relatie tot de daarop volgende methaanvorming"

(56) References cited:
JOURNAL OF WATER POLLUTION CONTROL FEDERATION, vol. 39, no. 8, 1967 pages 1369—1373 Washington D.C., U.S.A. J. W. MASSELLI et al.: "Sulfide Saturation for Better Digester Performance"

LES EAUX RESIDUAIRES INDUSTRIELLES-Meinck, Stooff & Kohlschütter, Masson (Paris) 1977, 2nd Edition, pp. 161—164

A process and apparatus for anaerobic conversion of organic material in an aqueous medium

The invention relates to a process for anaerobic conversion of organic material in an aqueous medium by fermentation in a fermentation region, comprising discharge of fermentation gas and fermented waste water and precipitation of sludge.

Such a method is known from "Les eaux résiduaires industrielles" Meinck, Stoff & Kohl-schütter, published by Masson (Paris) 1977, 2nd edition pages 161 to 164.

This process as used for purifying waste water provides on the one hand a valuable and non-polluting gas in the form of methane, while on the other hand a sludge is produced which may be used as fodder, as this sludge is comparable with one-cell proteins.

Unfortunately this known anaerobic purification process involves rather long residence times of waste water to be purified in the devices as used for such a process.

In order to reduce the minimum residence times of waste water to be purified by an anaerobic purification by a maximum factor of 100, a two-stage system, comprising a bacterial acidification stage and a fermentation stage during which the final purification is performed, has been proposed in DE INGENIEUR, vol. 90 no. 40 October 1978 KIVI pages 755 to 757.

This bacterial acidification is a rapidly proceeding process having a rate of about 1 kg chemical oxygen demand (COD)/kg sludge/h at a bacteria-concentration of 5% i.e. 50 kg COD/m³/h at 35°C and of 70 kg COD/m³/h at a temperature of 50°C.

For substrates such as proteins which are difficult to hydrolyse, said velocities are about 10 times lower. The conversion of lignocellulose complexes, however, proceeds optimally at a temperature of about 50°C.

At any rate sugars and starch forming the main environmental pollution problems are rapidly converted by means of such methods.

Such a bacterial acidification stage offers the disadvantage, however, that during bacterial acidification, the acidity decreases too much, so that this acidification does not proceed in an optimal way.

In the second or fermentation stage of the process as mentioned hereinbefore, the methane fermentation proceeds in a methane-fermentation reactor, the methane-forming-sludge being regularly recovered from the lower side of the methane fermentation vessel, said sludge being used as initiator in other reactors, while from the upper side of the reactor the formed methane and the purified waste water are discharged.

This method presents the disadvantage that during the anaerobic purification the liquid in the reactor will be stirred vigorously by the gas formed during said anaerobic fermentation which causes the bacteria-concentration to be equal at any location in the reactor and therefore also at the discharge opening of the reactor. Consequently too much valuable bacterial material will be entrained with the discharged liquid and will get lost.

So as to remedy said disadvantage it has been suggested (vide DE—A—27 28 585) to form a precipitation room in the upper side of the fermentation vessel through which a liquid stream is passed, flowing from the lower side towards the upper side, thus enabling sludge to precipitate. In practice these measures have, however, proved to be in-sufficient.

The present invention now aims to provide a method and device in which the above-mentioned disadvantages are fully overcome.

This object is attained according to the invention in that the aqueous medium to be purified is passed as a continuous stream through a plurality of successive precipitation zones, the produced sludge being discharged from each precipitation zone and the fermentation gas formed in the precipitation zones, is conveyed into an organic material-containing aqueous medium mass situated in a region beside said precipitation zones.

The fermentation gas from the precipitation zones together with other fermentation gas from the aqueous medium mass now strongly stirs the organic material containing aqueous medium mass, supplied to the fermentation region or to the fermentation vessel, which implies that the residence time of the waste liquid in the fermentation vessel may be considerably reduced.

Moreover the separation of sludge from fermented liquid to be discharged is considerably increased so that the waste water discharged after the purification will hardly contain any sludge and the sludge thus produced during anaerobic purification, will flow from the precipitation spaces back into the fermentation vessel, in a very valuable state.

It should be noted that according to FR—A—376 442 waste water, an aqueous medium to be purified, may be passed through a septic tank comprising a plurality of zones or compartments, the fermentation gas as formed in each zone or compartment escapes and the produced sludge is recovered in each zone or compartment.

The aqueous medium to be purified may be advantageously subjected to a bacterial acidification before the fermentation region or the fermentation vessel, while controlling the pH, which is appropriately performed by the addition of an acid neutralizing agent, particularly calcium carbonate, during the bacterial acidification process. Consequently a neutral pH within the limits of 5,5 to 8 may be maintained during the anaerobic fermentation. The addition of calcium carbonate further offers the advantage that calcium-ammonium-phosphate precipitates from waste liquids having a high phosphate content.

The process in accordance with the invention provides a maximum bacteria concentration both in the bacterial acidification stage and in the fermentation stage.

The present invention also relates to an apparatus for anaerobic conversion of organic material in an aqueous medium, comprising a fermentation vessel, provided with at least one fermentation-vessel inlet, a fermentation-vessel-outlet, a sludge discharge, a main gas outlet for discharging gas from the fermentation vessel, and at least one precipitation space in connection with the fermentation-vessel-outlet, being characterized in that the fermentation vessel comprises a plurality of successive precipitation spaces for the passing of a continuous stream of liquid, at least some of said precipitation spaces comprising a precipitation-space-gas-outlet and a precipitation-space-sludge-outlet, the precipitation-space-gas-outlet debouching through gas locks, into a first fermentation-vessel-space, situated beside the precipitation spaces, and a fermentation-vessel-inlet debouching into said first fermentation vessel space.

The fermentation vessel inlet is appropriately connected with a bacterial acidification-vessel-outlet of a bacterial acidification vessel. Such a bacterial acidification vessel is conveniently provided with a pH measuring device for determining the pH, which pH measuring device acts upon a member for selectively controlling the supply of an acid neutralizing agent, particularly calcium carbonate, to the bacterial acidification vessel.

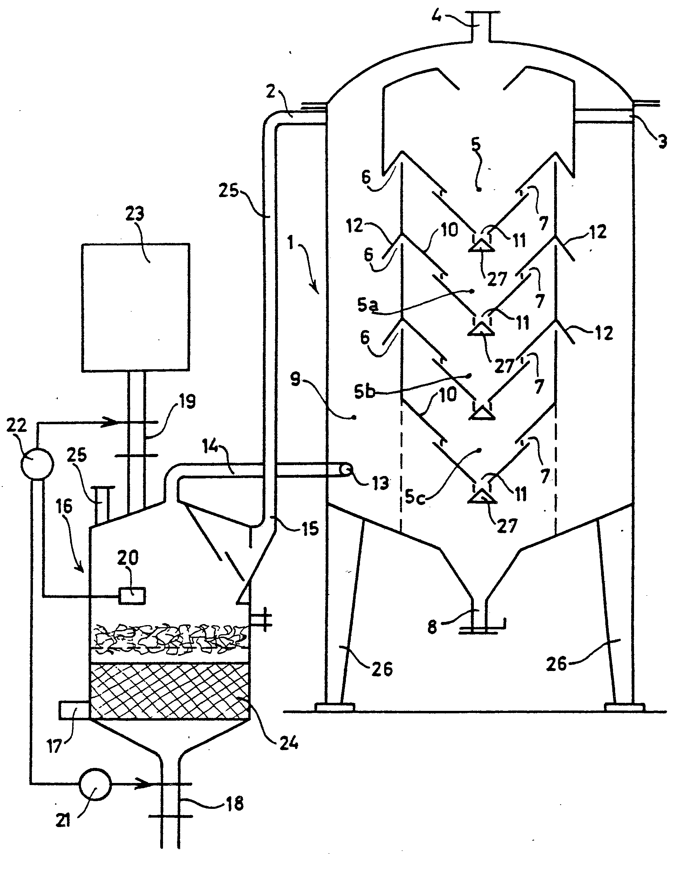

The present invention will be illustrated with respect to an embodiment in the drawing which schematically shows an apparatus in accordance with the invention.

The drawing shows a fermentation vessel 1 being provided with at least one fermentation-vessel-inlet 2, a fermentation-vessel-outlet 3, a sludge discharge 8 and a main gas discharge 4 for the discharge of gas from the fermentation vessel 1. The fermentation-vessel-outlet 3 is connected with a precipitation space 5 which is located within the vessel 1.

In order to obtain a continuous supply of organic material (to be subjected to an anaerobic fermentation) and of an aqueous medium, such as waste water, to the fermentation vessel 1, said fermentation vessel 1 is provided with a plurality of equidistant fermentation-vessel-inlets 2 extending around the circumference of said vessel. Said inlets may debouch at various different levels.

Equidistantly circumferentially located fermentation-vessel-outlets 3 may also be provided in dependence of the dimensions of the apparatus according to the invention.

The fermentation vessel 1 in accordance with the invention comprises a plurality of successive precipitation spaces 5, 5a, 5b, 5c, through which liquids may be passed in a continuous stream, each precipitation space 5, 5a, 5b, 5c being provided with a precipitation space-gas-discharge 6 and a precipitation-space-sludge-discharge 11.

The precipitation spaces 5, 5a, 5b, 5c, are superimposed within the fermentation vessel 1.

The precipitation-space-gas-discharges 6 debouch via gas locks into a first fermentation-vessel-space 9 located beside the precipitation spaces 5, 5a, 5b, 5c, into which space 9 debouche fermentation-vessel-inlets 2.

The first fermentation-vessel-space 9 is arranged concentrically around the precipitation spaces and is connected therewith via a liquid supply 7, located at the lower side of the precipitation space 5c. The other precipitation spaces 5b, 5a and 5 are also provided with liquid supplies 7.

In order to obtain the gas locks for discharging the gas as formed in the precipitation spaces 5, 5a, 5b, 5c each precipitation space is provided with gas guiding partitions 12 each in the form of a circular wall, which inclines towards the base of the fermentation vessel 1.

It has been found that in a fermentation vessel according to the invention an optimum anaerobic fermentation can be provided at bacteria concentrations ranging from about 8% to 10%.

Gas spreading means 27 situated below the sludge discharges 11 prevent gas produced in a precipitation space 5, 5a, 5b and 5c, escaping through said sludge-discharges 11, towards a precipitation space situated above, the latter discharge(s).

The fermentation vessel may be mounted upon legs 26 in order to discharge sludge through a sludge-discharge 8 being provided with a closing valve. It will be obvious, however, that large vessels may also be located directly upon a foundation and that their sides may comprise a methane-fermentation-sludge outlet.

The main gas discharge 4 is located at the uppermost point of the fermentation vessel 1.

The fermentation vessel inlets 2 may be connected with a bacterial acidification-vessel-discharge 15 through a line 25, being integral with a bacterial acidification vessel 16. Said bacterial acidification vessel 16 comprises an inlet 17 located at the lower side of the acidification vessel, a sludge-discharge-lock 18, and a lock 19 for the supply of acid neutralizing agent, particularly calcium carbonate from a calcium-carbonate-storage vessel 23.

So as to optimally control the pH in the bacterial acidification vessel 16, said acidification vessel 16 comprises a pH measuring-control member 20, cooperating with a control member 22 acting upon the valve 19 and thus controlling the supply of calcium carbonate to the acidification vessel. The member 22 also controls the sludge discharge in lock 18, thus preventing unreacted calcium carbonate to be discharged simultaneously with the sludge.

The bacteria concentration in the bacterial acidification vessel amounts to e.g. 6%.

A gas discharge line 14 debouching into the first fermentation-vessel-space 9 through a mouth 13, may supply gas produced in the bacterial acidification vessel 16, to said first fermentation-vessel-space 9.

Through an inlet 25 the bacterial acidification vessel may be supplied with compounds for the removal of heavy metals, in the form of sulfides, from the waste liquids. In case of excess of heavy metals in the waste liquid, calcium-sulfate is supplied via said inlet 25; in case of excess sulfides in the waste liquid, the removal of heavy metals in the form of sulfides may occur by adding iron ions or calcium carbonate.

Preferably such heavy metals are removed in a prior processing stage, so that the sulfides are obtained in a concentrated form.

Very high conversion rates are obtained by means of the two-stage anaerobic purification process as described hereinbefore, so that large amounts of waste liquids may be purified in very small installations in a rapid and efficient manner.

In order to purify waste liquids one proceeds, e.g., as follows:

A sludge layer 24 consisting of bacteria to be used for anaerobic purification, is disposed in the bacterial acidification vessel 16.

The pH of the liquid is measured by the pH measuring means 20 during the supply of waste water to be purified through bacterial acidification-vessel-inlet 17. Said pH measuring means 20 acts upon a controlling means 22 which controls a valve 19 for supplying calcium carbonate from calcium carbonate storage vessel 23.

A very selective and exact pH control can be obtained in the manner as described above.

Sludge produced during the acidification may be discharged through lock 18 representing the bacterial acidification-vessel-sludge-outlet. This lock 18 is also controlled by the controlling means 22 through an operating member 21.

From the bacterial acidification vessel 16 the waste water flows through acidification-vessel-liquid-discharge 15 and line 25, towards the fermentation-vessel-inlets 2, preferably four, located equidistantly around the circumference of the fermentation vessel 1.

Said waste liquid arrives in the first fermentation-vessel-space 9 where it is stirred vigorously by means of gas being introduced into said waste liquid through gas locks 6, said stirring taking place in addition to the stirring already occurring in space 9 by gas produced during anaerobic fermentation. The latter gas emanates from the separate successive precipitation spaces 5c, 5b, 5a, 5. Liquid supplied to each of said precipitations spaces through a liquid inlet 7 is allowed to precipitate, whereby the produced sludge may be discharged through a sludge discharge 11, while gas, as mentioned hereinbefore, is discharged via gas lock 6.

A very homogeneous bacteria concentration is thus obtained in the annular first fermentation-vessel-space 9, so that an optimum fermentation takes place.

On the other hand stirring in these spaces may be improved by conveying methane gas from the bacterial acidification vessel to the first fermentation-space 9, through a line 14 having a mouth 13.

Gas escaping via gas locks 6, said gas vigorously stirring the liquid in the annular space 9, as described hereinbefore is discharged via gas outlet 4; treated waste liquid, almost without sludge, disappears via fermentation-vessel-outlet 3, which debouches into the uppermost precipitation space 5.

The use of successive and superimposed precipitation spaces 5c, 5b, 5a, 5 on the one hand and the annular space 9 surrounding said precipitation spaces on the other hand promotes a very high bacteria concentration, involving a high anaerobic fermentation, so that small residence times in the fermentation vessel 1 suffice.

In order to remove heavy metals from the waste liquids it is recommended to precipitate same in the form of metal sulfides. Excess of heavy metals may be removed by adding calcium sulfate; when, however, the liquid contains excess sulfates, the heavy metals may be removed by adding iron ions or calcium carbonate. Said additions may occur through inlet 25 but a separation of heavy metal sulfides in waste liquid is preferably performed in a separate stage, so that said harmful sulfides are obtained in a concentrated form.

The minimal size of the precipitation spaces in the fermentation vessel are determined by the allowable surface charge, amounting at most to the precipitation velocity of the sludge or less, in $m^3$ supplied waste liquid p/$m^2$ precipitation space surface/h.

The pH measuring device 20 also controls, through the controlling means 22, an operating member 21 for the lock 18, wherethrough the sludge 24 from the acidification vessel is discharged.

Both during the bacterial acidification and the methane fermentation a bacteria mixture should preferably be applied.

Although the drawing shows that the precipitation spaces are centrally disposed in the fermentation vessel, it will be obvious that said spaces may also be disposed at the outside of the fermentation vessel while the first fermentation-vessel-space 9 may then be centrally disposed.

Claims

1. A process for anaerobic conversion of organic material in an aqueous medium by fermentation in a fermentation region, comprising discharge of fermentation gas and fermented waste water and precipitation of sludge, characterized in that the aqueous medium to be purified is passed as a continuous stream through a plurality of successive precipitation zones the sludge is discharged from each precipitation zone and the fermentation gas formed in the precipitation zones is conveyed into an organic material containing aqueous medium mass, situated in a region beside said precipitation zones.

2. A process according to claim 1, characterized in that aqueous medium to be purified is subjected to bacterial acidification before fermentation, under selective control of the pH, said pH being controlled during the bacterial acidification by the addition of an acid neutralizing agent.

3. A process according to claim 1 or 2, characterized in that during the fermentation the pH is substantially maintained at neutral values.

4. A process according to claims 1 to 3, characterized in that heavy metals are removed from the aqueous medium, as metal sulfides, said sulfides being removed from the aqueous medium during a processing stage, preceding fermentation or bacterial acidification.

5. A process according to claim 4, characterized in that heavy metal sulfides are removed by adding calcium sulfate if heavy metals are in excess or by adding iron ions or calcium carbonate if sulfates are in excess.

6. A process according to claims 1—5, characterized in that gas emanating from the bacterial acidification is passed into an organic material containing aqueous medium mass situated beside the precipitation zones (5, 5a, 5b, 5c).

7. An apparatus for anaerobic conversion of organic material in an aqueous medium, comprising a fermentation vessel provided with at least one fermentation-vessel-inlet (2), a fermentation-vessel-outlet (3), a sludge discharge (8), a main gas outlet (4) for discharging gas from the fermentation vessel, and at least one precipitation space in connection with the fermentation-vessel-outlet (3), characterized in that the fermentation vessel comprises a plurality of successive precipitation spaces (5, 5a, 5b, 5c) for the passing of a continuous stream of liquid, at least some of said precipitation spaces comprising a precipitation space-gas-outlet (6) and a precipitation-space-sludge-outlet (11), the precipitation-space-gas-outlets (6) debouch through gas locks, into a first fermentation-vessel-space (9), situated beside the precipitation spaces (5, 5a, 5b, 5c), and a fermentation-vessel-inlet (2) debouching into said first fermentation-vessel-space (9).

8. An apparatus according to claim 7, characterized in that the precipitation spaces (5, 5a, 5b, 5c) are superimposed in the fermentation vessel and the first fermentation-vessel-space (9) is arranged concentrically around the precipitation spaces (5, 5a, 5b, 5c).

9. An apparatus according to claim 7 or 8, characterized in that the precipitation zones (5, 5a, 5b, 5c) consist of a housing connected with a funnel-shaped bottom (10) which is provided with a sludge-discharge (11) and a precipitation-space-liquid-inlet (7).

10. An apparatus according to claims 7—9, characterized in that the precipitation spaces (5, 5a, 5b, 5c) are provided with gas-guiding-partitions (12) extending into the first fermentation-vessel-space (9), said gas-guiding-partitions (12) forming a circular wall which inclines towards the bottom of the fermentation vessel.

11. An apparatus according to claims 7—10, characterized in that the fermentation vessel comprises a plurality of fermentation-vessel-inlets (2) located equidistantly around the circumference of said fermentation vessel.

12. An apparatus according to claims 7—11 comprising a bacterial acidification vessel (16), characterized in that the main gas outlet (4) is situated in the uppermost part of the fermentation vessel and a gas inlet (13) is connected with a bacterial acidification-vessel-gas-outlet (14).

13. An apparatus according to claim 12, characterized in that the bacterial acidification vessel (16) comprises an acidification-vessel-inlet (17) and an acidification-vessel-sludge outlet (18), said bacterial acidification vessel being provided with at least one acid-neutralizing-agent-inlet (19).

14. An apparatus according to claim 13, characterized in that the bacterial acidification vessel (16) comprises a pH measuring device (20), said pH measuring device being provided with a control means (22) which governs the supply of acid neutralizing agent and/or the discharge of sludge.

**Revendications**

1. Procédé pour la conversion anaérobie de matière organique dans un milieu aqueux par fermentation dans une région de fermentation comprenant la décharge du gaz de fermentation et de l'eau résiduaire fermentée et la précipitation de la boue, caractérisé en ce que l'on fait passer le milieu aqueux à purifier en un courant continu à travers plusieurs zones successives de précipitation, la boue produite est déchargée de chaque zone de précipitation et le gaz de fermentation formé dans les zones de précipitation est transporté dans une masse de milieu aqueux contenant la matière organique, située dans une région à côté desdites zones de précipitation.

2. Procédé selon la revendication 1, caractérisé en ce que le milieu aqueux à purifier est soumis à l'acidification bactérienne avant fermentation, avec réglage sélectif du pH, ledit pH étant réglé pendant l'acidification bactérienne par l'addition d'un agent neutralisant les acides.

3. Procédé selon la revendication 1 ou 2, caractérisé en ce que pendant la fermentation le pH est sensiblement maintenu à des valeurs neutres.

4. Procédé selon les revendications 1 à 3, caractérisé en ce que les métaux lourds sont éliminés du milieu aqueux sous forme de sulfures métalliques, lesdits sulfures étant éliminés du milieu aqueux pendant une étape de traitement précédant la fermentation ou l'acidification bactérienne.

5. Procédé selon la revendication 4, caractérisé en ce que les sulfures de métaux lourds sont éliminés par addition de sulfate der calcium si les métaux lourds sont en excès ou par addition d'ions fer ou de carbonate de calcium si les sulfates sont en excès.

6. Procédé selon les revendications 1 à 5, caractérisé en ce que l'on fait passer le gaz sortant de l'acidification bactérienne dans une masse de milieu aqueux contenant la matière organique située à côté des zones de précipitation (5, 5a, 5b, 5c).

7. Appareil pour la conversion de matière organique dans un milieu aqueux, comprenant un récipient de fermentation muni d'au moins un orifice d'entrée (2) du récipient de fermentation, un orifice de sortie (3) du récipient de fermentation, une décharge de boue (8), un orifice principal de sortie de gaz (4) pour la décharge de gaz du récipient de fermentation et d'au moins un espace de précipitation relié avec l'orifice de sortie (3) du récipient de fermentation, caractérisé en ce que le récipient de fermentation comprend plusieurs espaces successifs de précipitation (5, 5a, 5b, 5c) pour le passage d'un courant continu de liquide, quelques-uns au moins desdits espaces de précipitation comprenant un orifice de sortie de gaz (6) de l'espace de précipitation et un orifice de sortie de la boue (11) de l'espace de précipitation les orifices de sortie de gas (6) des espaces de précipitation débouchant à travers des matelas de gaz dans un premier espace (9) du récipient de fermentation, situé à côté des espaces de précipitation (5, 5a, 5b, 5c) et l'orifice d'entrée (2) du récipient de fermentation débouchant dans ledit premier espace (9) du récipient de fermentation.

8. Appareil selon la revendication 7, caractérisé en ce que les espaces de précipitation (5, 5a, 5b, 5c) sont superposés dans le récipient de fermentation et le premier espace (9) du récipient de fermentation est disposé concentriquement autour des espaces de précipitation (5, 5a, 5b, 5c).

9. Appareil selon la revendication 7 ou 8, caractérisé en ce que les zones de précipitation (5, 5a, 5b, 5c) consistent en un logement relié à un fond en entonnoir (10) qui est muni d'une décharge de boue (11) et d'un orifice d'entrée de liquide (7) de l'espace de précipitation.

10. Appareil selon les revendications 7 à 9, caractérisé en ce que les espaces de précipitation (5, 5a, 5b, 5c) sont munis de cloisons de guidage du gaz (12) se prolongeant dans le premier espace (9) du récipient de fermentation, lesdites cloisons de guidage (12) formant une paroi circulaire qui s'incline vers le fond du récipient de fermentation.

11. Appareil selon les revendications 7 à 10, caractérisé en ce que le récipient de fermentation comprend plusieurs orifices d'entrée (2) du récipient de fermentation équidistants sur la périphérie dudit récipient de fermentation.

12. Appareil selon les revendications 7 à 11, comprenant un récipient d'acidification bactérienne (16), caractérisé en ce que l'orifice principal de sortie de gaz (4) est situé à la partie supérieure du récipient de fermentation et un orifice d'entrée de gaz (13) est relié à un orifice de sortie de gaz (14) du récipient l'acidification bactérienne.

13. Appareil selon la revendication 12, caractérisé en ce que le récipient d'acidification bactérienne (16) comprend un orifice d'entrée (17) du récipient d'acidification et un orifice de sortie (18) du récipient d'acidification, ledit récipient d'acidification bactérienne étant muni d'au moins un orifice d'entrée (19) de l'agent neutralisant les acides.

14. Appareil selon la revendication 13, caractérisé en ce que le récipient d'acidification bactérienne (16) comprend un dispositif de mesure du pH (20), ledit dispositif de mesure du pH étant muni d'organes de réglage qui règlent l'alimentation en agent neutralisant les acides et/ou la décharge de boue.

## Patentansprüche

1. Verfahren zur anaeroben Umwandlung von organischem Material in einem wäßrigen Medium durch Fermentierung in einem Fermentationsbereich, wobei man ein Fermentationsgas und fermentiertes Abwasser abläßt und Schlamm ausfällt, dadurch gekennzeichnet, daß das zu reinigende wäßrige Medium als ein kontinuierlicher Strom durch mehrere aufeinander folgende Ausfällzonen geführt wird, der Schlamm aus jeder Ausfällzone abgelassen wird und das in den Ausfällzonen gebildete Fermentationsgas in eim organisches Material enthaltende wäßrige Medium-Masse, die in einem Bereich neben den genannten Ausfällzonen angeordnet ist, geleitet wird.

2. Verfahren nach Anspruch 1, dadurch gekennzeichnet, daß das zu reinigende wäßrige Medium vor der Fermentierung einer bakteriellen Ansäuerung unter selektiver Steuerung des pH unterworfen wird, wobei der pH während der bakteriellen Ansäuerung durch Zugabe eines sauren Neutralisierungsmittels gesteuert wird.

3. Verfahren nach Anspruch 1 oder 2, dadurch gekennzeichnet, daß während der Fermentierung der pH im wesentlichen bei neutralen Werten gehalten wird.

4. Verfahren nach den Ansprüchen 1 bis 3, dadurch gekennzeichnet, daß Schwermetalle aus dem wäßrigen Medium als Metallsulfide entfernt werden, wobei die Sulfide aus dem wäßrigen Medium während einer Verarbeitungsstufe, vorangegangener Fermentierung oder bakteriellen Ansäuerung entfernt werden.

5. Verfahren nach Anspruch 4, dadurch gekennzeichnet, daß die Schwermetallsulfide durch Zugabe von Kalziumsulfat, wenn die Schwermetalle im Überschuß vorliegen, oder durch Zugabe von Eisenionen oder Kalziumcarbonat, wenn die Sulfate im Überschuß vorliegen, entfernt werden.

6. Verfahren nach einem der Ansprüche 1 bis 5, dadurch gekennzeichnet, daß Gas, das bei der bakteriellen Ansäuerung entsteht, in eine wäßrige Masse enthaltendes organisches Material geleitet wird, welche neben den Ausfällzonen (5, 5a, 5b, 5c) angeordnet ist.

7. Vorrichtung für die anaerobe Umwandlung von organischem Material in einem wäßrigem Medium, enthaltend ein Fermentationsgefäß mit wenigstens einem Fermentationsgefäßeinlaß (2), einem Fermentationsgefäßauslaß (3), einem Schlammablaß (8), einem Gashauptauslaß (4) zum Ableiten des Gases von dem Fermentationsgefäß und wenigstens einem Ausfällraum in Verbindung mit dem Fermentationsgefäßauslaß (3), dadurch gekennzeichnet, daß das Fermentationsgefäß mehrere aufeinanderfolgende Ausfällräume (5, 5a, 5b, 5c) für das Hindurchleiten eines kontinuierlichen Stroms von Flüssigkeit besitzt, wobei wenigstens einige Ausfällräume einen Auslaß (6) für das Ausfällraumgas und einen Auslaß (11) für den Ausfällraumschlamm besitzt, die Auslässe (6) für das Ausfällgas durch Gasabsperrungen in einen ersten Fermentationsgefäßraum (9) einmünden, der neben den Ausfällräumen (5, 5a, 5b, 5c) angeordnet ist, und ein Fermentationsgefäßeinlaß (2) in den genannten ersten Fermentationsgefäßraum (9) einmündet.

8. Vorrichtung nach Anspruch 7, dadurch gekennzeichnet, daß die Ausfällräume (5, 5a, 5b, 5c) in dem Fermentationsgefäß übereinander liegen, und daß der erste Fermentationsgefäßraum (9) konzentrisch um die Ausfällräume (5, 5a, 5b, 5c) angeordnet ist.

9. Vorrichtung nach Anspruch 7 oder 8, dadurch gekennzeichnet, daß die Ausfällzonen (5, 5a, 5b, 5c) aus einem Gehäuse bestehen, das mit einem trichterförmigen Boden (10) verbunden ist, der einen Schlammablaß (11) und einen Einlaß (7) für die Ausfällraumflüssigkeit besitzt.

10. Vorrichtung nach den Ansprüchen 7 bis 9, dadurch gekennzeichnet, daß die Ausfällräume (5, 5a, 5b, 5c) Gasleitwände (12) besitzen, die sich in den ersten Fermentationsgefäßraum (9) erstrecken, wobei die Gasleitwände (12) eine ringförmige Wand bilden, die nach dem Boden des Fermentationsgefäßes geneigt ist.

11. Vorrichtung nach den Ansprüchen 7 bis 10, dadurch gekennzeichnet, daß das Fermentationsgefäß mehrere Fermentationsgefäßeinlässe (2) besitzt, die in gleichen Abständen um den Umfang des Fermentationsgefäßes herum angeordnet sind.

12. Vorrichtung nach den Ansprüchen 7 bis 10, enthaltend ein Gefäß für die bakterielle Ansäuerung, dadurch gekennzeichnet, daß der Hauptgasauslaß (4) im obersten Teil des Fermentationsgefäßes angeordnet ist und ein Gaseinlaß (13) mit einem Gasauslaß (14) für das Gefäß für die bakterielle Ansäuerung verbunden ist.

13. Vorrichtung nach Anspruch 12, dadurch gekennzeichnet, daß das Gefäß (16) für die bakterielle Ansäuerung einen Ansäuerungsgefäßeinlaß (17) und einen Ansäuerungsgefäßauslaß (18) aufweist, wobei das Gefäß für die bakterielle Ansäuerung wenigstens einen Einlaß (19) für ein Säure neutralisierendes Mittel besitzt.

14. Vorrichtung nach Anspruch 13, dadurch gekennzeichnet, daß das bakterielle Ansäuerungsgefäß (16) eine pH-Meßvorrichtung (20) aufweist, wobei die pH-Meßvorrichtung eine Steuerungsvorrichtung (22) besitzt, die die Zufuhr von Säure neutralisierendem Mittel und/oder den Schlammablaß regelt.